# EUROPEAN PATENT APPLICATION

(11) **EP 4 491 745 A1**
(43) Date of publication of application: **15.01.2025**
(21) Application number: 23767176.3
(22) Date of filing: 08.03.2023
(51) Int. Cl.: C12Q 1/6886, C12N 9/10

(54) **PHARMACEUTICAL COMPOSITION COMPRISING SETMAR INHIBITOR FOR PREVENTING OR TREATING CANCER**

(30) Priority: 08.03.2022 KR 20220029596
(71) Applicant: Korea Advanced Institute of Science and Technology, Daejeon 34141 (KR)
(72) Inventor: CHO, Kwang Hyun, Daejeon 34141 (KR); GONG, Jeong Ryeol, Daejeon 34141 (KR); KIM, Ju Hee, Daejeon 34141 (KR)
(74) Representative: Potter Clarkson
(86) International application number: PCT/KR2023/003198
(87) International publication number: WO 2023/172075

(57) **Abstract**

The present invention relates to a composition comprising a SETMAR inhibitor for preventing or treating cancer. It has been identified that if SETMAR is inhibited in cancer cells, carcinogenesis reversion, which is the differentiation of cancer cells into normal cells, is successfully achieved, and thus, unlike conventional anticancer agents that simply induce cancer cell death, the present invention can be effectively used as a treatment method excluding the side effect of normal cell death that can occur during anticancer treatment, and converting cancer cells into normal cells.

## Description

### [Technical Field]

The present invention relates to a composition including a SETMAR inhibitor for preventing or treating cancer, which is characterized to reverse cancer cells into normal cells.

### [Background Art]

Cancer is one of incurable diseases that humanity has to solve, and a huge amount of capital is being invested in development to cure the cancer around the world. In Korea, the cancer is the number one cause of death due to disease, in which about more than 100,000 people are diagnosed every year, and about more than 60,000 people have died. Among them, liver cancer is identified as a major cause of cancer death according to the "Global cancer statistics 2020" report published by the International Agency for Research on Cancer (IARC) in 2020.

Carcinogens as cause factors of such cancer include smoking, ultraviolet rays, chemicals, food, and other environmental factors. However, the causes are diverse, so that it is difficult to develop therapeutic agents, and the effectiveness of the therapeutic agents is also different depending on an occurring site. Substances currently used as the therapeutic agents have significant toxicity and do not selectively remove cancer cells, so that there is an urgent need to develop less toxic and effective anticancer agents to not only treat cancer after development, but also prevent the development of cancer.

The primary treatment principle for cancer is surgical resection, but since there is a high recurrence rate even after surgical resection, auxiliary treatment such as radiotherapy or chemotherapy is required to prolong survival duration, alleviate symptoms, and maintain and improve the quality of life. However, there are no absolute principles regarding the type and route of administration of drugs in chemotherapy, and the effectiveness is also not satisfactory. In addition, even among patients with the same cancer, there are significant differences in response rate and survival probability to chemotherapy. To date, active research has been conducted on drugs targeting the tumor's genetic predisposition, particularly growth signaling transduction, and a microenvironment of tumor cells, to develop therapeutic agents for patients with solid cancers, but satisfactory therapeutic agents have not been developed.

In particular, it is known that the activity of epigenetic regulators is required along with the accumulation of mutations in the carcinogenesis of converting normal cells into cancer cells. Therefore, in order to achieve excellent therapeutic activity against cancer, if cancer cells may be reversed into normal cells or normal-like cells through such an epigenetic approach, the method could be an effective new cancer treatment, which will contribute to the realization of personalized medicine, improving patients' survival rate and quality of life reduced by the treatment with unnecessary anticancer agents through targeted treatment.

### [Disclosure]

### [Technical Problem]

Under this background, the present inventors have made extensive research efforts to develop a method for treating cancer safely and effectively without side effects. As a result, the present inventors found that when a SETMAR gene known as an epigenetic regulator was inhibited, cancer cells may be reversed into normal cells, thereby achieving an excellent cancer treatment (remission) effect, while discovering key factors necessary for reversing cancer cells into normal cells (or normal-like cells) at a pan-cancer level based on data produced by various technologies for confirming epigenetic changes, and then completed the present invention.

An object of the present invention is to provide a pharmaceutical composition for preventing or treating cancer.

Another object of the present invention is to provide an anticancer adjuvant.

Yet another object of the present invention is to provide a food composition for preventing or alleviating cancer.

Still another object of the present invention is to provide a composition for converting cancer cells into normal cells or normal-like cells.

Still another object of the present invention is to provide a method for inducing conversion of cancer cells into normal cells or normal-like cells.

Still another object of the present invention is to provide a screening method of a cancer therapeutic agent.

Still another object of the present invention is to provide a screening method of an agent capable of converting cancer cells into normal cells or normal-like cells.

Still another object of the present invention is to provide a method for treating cancer.

### [Technical Solution]

The terms used herein are used for the purpose of description only and should not be construed to be limited. A singular expression includes a plural expression unless the context clearly indicates otherwise. In the present specification, it should be understood that the term "including" or "having" indicates that a feature, a number, a step, an operation, a component, a part or the combination thereof described in the specification is present but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art and are not interpreted as ideal or excessively formal meanings unless otherwise defined in the present invention.

Hereinafter, the present invention will be described in more detail.

According to an aspect of the present invention, the present invention provides a pharmaceutical composition for preventing or treating cancer, including a SET Domain and Mariner Transposase Fusion Gene (SETMAR) inhibitor.

The SETMAR of the present invention is a target gene which was discovered based on genome, epigenome, and transcriptome databases TCGA, ENCODE, GTEX, HPA, and FANTOM of various cancers and normal tissues and derived from 8,000 or more samples derived from 11 types of cancer, and can be used for various types of cancer. More specifically, the SETMAR is derived as a key regulator of leading reversion to normal cells, as a result of analyzing epigenetic regulators at a pan-cancer level based on histone modification, which may be an effective classification standard between normal cells and cancer cells derived from various tissues, through data analysis of gene regulatory networks. Therefore, the present invention can be used in various cancer types, including liver cancer, colon cancer, lung cancer, and kidney cancer cells.

The SETMAR inhibitor of the present invention may use any agent or means known in the art, as long as the inhibitor may reduce the SETMAR expression level or activity in cancer cells, as the purpose of the present invention.

Preferably, the SETMAR inhibitor may be at least one selected from the group consisting of antisense oligonucleotide, small interference RNA (siRNA), short hairpin RNA (shRNA), microRNA (miRNA), and ribozyme that bind complementarily to mRNA of the SETMAR gene; or at least one selected from the group consisting of a compound, a peptide, a peptide mimetic, a substrate analog, an aptamer, and an antibody that specifically bind to the SETMAR protein, but is not limited thereto.

As used herein, the term "antisense nucleic acid" refers to DNA, RNA, or derivatives thereof containing a nucleic acid sequence complementary to a specific mRNA sequence, and serves to inhibit the translation to a protein of mRNA by binding to the complementary sequence in mRNA. The antisense sequence refers to a DNA or RNA sequence complementary to mRNA of the gene and capable of binding to the mRNA, and may inhibit translation of the mRNA, translocation into the cytoplasm, maturation, or all other essential activities for overall biological functions.

In addition, the antisense nucleic acid may be modified at one or more base, sugar or backbone positions to enhance the efficacy. The nucleic acid backbone may be modified with phosphorothioate, phosphotriester, methyl phosphonate, short-chain alkyl, cycloalkyl, short-chain heteroatomic, heterocyclic intersugar linkages, and the like. In addition, the antisense nucleic acid may include one or more substituted sugar moieties. The antisense nucleic acid may include modified bases. The modified bases include hypoxanthine, 6-methyladenine, 5-methylpyrimidine (particularly, 5-methylcytosine), 5-hydroxymethylcytosine (HMC), glycosyl HMC, gentobiosyl HMC, 2-aminoadenine, 2-thio uracil, 2-thiothymine, 5-bromouracil, 5-hydroxymethyluracil, 8-azaguanine, 7-deazaguanine, N6(6-aminohexyl)adenine, 2,6-diaminopurine, and the like. In addition, the antisense nucleic acid may chemically bind to one or more moieties or conjugates that improve the activity and cell adhesion of the antisense nucleic acid. The antisense nucleic acid includes fat-soluble moieties, such as cholesterol moiety, cholesteryl moiety, cholic acid, thioether, thiocholesterol, fatty chain, phospholipid, polyamine, polyethylene glycol chain, adamantane acetic acid, palmityl moiety, octadecylamine, hexylaminocarbonyl-oxycol esterol moiety, and the like, but is not limited thereto. The antisense oligonucleotide may be synthesized *in vitro* by a conventional method to be administered *in vivo,* or may be synthesized *in vivo.*

As used herein, the "siRNA" means a nucleic acid molecule capable of mediating RNA interference or gene silencing. Since the siRNA may inhibit the expression of a target gene, the siRNA is provided as an effective gene knock-down method or gene therapy method.

The siRNA molecule of the present invention may have a structure forming a double chain in which a sense strand (a sequence corresponding to an mRNA sequence of a SETMAR gene as a target gene) and an antisense strand (a complementary sequence to the mRNA sequence) are located opposite each other, and the siRNA molecule of the present invention may have a single-stranded structure with self-complementary sense and antisense strands. Furthermore, the siRNA is not limited to complete pairing of a double-stranded RNA portion paring RNAs, but may include a portion which is not paired by mismatch (corresponding bases are not complementary), bulge (there is no corresponding base on one chain), etc. In addition, when an siRNA end structure may suppress the expression of the target gene by an RNAi effect, both a blunt end and a cohesive end are possible, and the cohesive end structure is able to be both a 3'-end protruding structure and a 5'-end protruding structure.

The "shRNA" of the present invention is called small hairpin RNA or short hairpin RNA, and is used for silencing the gene by RNA interference. Usually, the shRNA is introduced into a target cell using a vector. Such an shRNA hairpin structure is also cleaved by other intracellular substances to become siRNA.

In an embodiment of the present invention, short hairpin RNA (shRNA) represented by SEQ ID NO: 1 was used as the SETMAR inhibitor, but it is not limited thereto as long as the purpose of the present invention can be achieved.

In addition, the present invention may include a functional equivalent of the shRNA base sequence represented by SEQ ID NO: 1. The "functional equivalent" refers to a polynucleotide that exhibits substantially the same physiological activity as the polynucleotide represented by the base sequence represented by SEQ ID NO: 1 by having a sequence homology of at least 70% or more, preferably 80% or more, more preferably 90% or more, much more preferably 95% or more, as a result of deletion, substitution or insertion of bases. The "% of sequence homology" with the polynucleotide is identified by comparing two optimally arranged sequences with a comparison region, and a part of a polynucleotide sequence in the comparison region may include addition or deletion (i.e., gap) compared to a reference sequence (without including addition or deletion) for an optimal alignment of the two sequences.

According to an embodiment of the present invention, when the SETMAR is inhibited, the conversion, that is, reversion of cancer cells into normal cells or normal-like cells is induced. In addition, the reversion into the normal cells or normal-like cells induces a change to normal cell shape, recovery of normal cell functions, or epigenetic changes to normal cells.

As used herein while mentioning the change of cancer cells into normal cells or normal-like cells, the terms "reversion" and "conversion" mean that cancer cells are differentiated into normal cells or normal-like cells when the SETMAR, an epigenetic regulator target gene in carcinogenesis discovered in the present invention, is inhibited in cancer cells, and are used interchangeably herein.

As used herein, the "normal-like cell" refers to a cell having a transcriptome of a cancer cell-derived normal cell, and refers to a normal cell, preferably a cell with the same or similar activity as or to the normal cell.

According to an embodiment of the present invention, the reversion into the normal cells or normal-like cells may induce a change to a normal cell shape, recovery of normal cell functions, or an epigenetic change to normal cells. In a preferred embodiment of the present invention, it was confirmed that when the SETMAR was inhibited in liver cancer cells, the reversion into the normal cells or normal-like cells was induced as in two liver cancer cell lines SNU475 and SNU761.

In addition, according to an embodiment of the present invention, it was confirmed that when the SETMAR was inhibited, at least one selected from the group consisting of proliferation ability, growth ability, metastatic ability, invasion ability, and migration ability of cancer cells was inhibited.

In addition, according to an embodiment of the present invention, it was confirmed that when the SETMAR was inhibited, the methylation of histones in cancer cells was increased or inhibited, and more specifically, the SETMAR inhibition increased trimethylation of histone H3 at lysine 4 (H3K4me3) or inhibited trimethylation of histone H3 at lysine 27 (H3K27me3).

Accordingly, it is verified that the SETMAR inhibition of the present invention may reprogram or convert cancer cells into normal cells or normal-like cells.

Unlike conventional anticancer agents that kill cancer cells, when the SETMAR inhibitor of the present invention was treated on cancer cells, the SETMAR inhibitor may inhibit cancer cell characteristics of target cells, such as proliferation ability, growth ability, metastatic ability, invasion ability, or migration ability, and promote the natural shape and function of the target cells, such as a change to a normal cell shape, recovery of normal cell functions or induction of epigenetic changes into normal cells, thereby exhibiting the same functions as normal cells by converting cancer cells into normal or normal-like cells again. Accordingly, unlike conventional anticancer agents that only induce cell death, it is possible to solve side effects caused by cell death.

In addition, the cancer of the present invention may include any cancer as long as the composition of the present invention achieves the desired effect. For example, the cancer may be at least one selected from the group consisting of liver cancer, colon cancer, lung cancer, adrenal cancer, stomach cancer, breast cancer, blood cancer, bone cancer, pancreatic cancer, skin cancer, head or neck cancer, cutaneous or intraocular melanoma, uterine sarcoma, ovarian cancer, rectal cancer, anal cancer, fallopian tube carcinoma, endometrial carcinoma, cervical cancer, small intestine cancer, endocrine cancer, thyroid cancer, parathyroid cancer, soft tissue tumor, urethral cancer, prostate cancer, bronchogenic cancer, and bone marrow tumor. In an embodiment of the present invention, the cancer targets liver cancer, but is not limited thereto.

As used herein, the term "including as the active ingredient" means including the SETMAR inhibitor, which is the active ingredient of the present invention, in an amount sufficient to achieve a predetermined effect or activity. The SETMAR inhibitor may be administered in a pharmaceutically effective amount, and the effective amount level may be determined depending on the type, age, and sex of a subject, sensitivity to a drug, a treatment period, drugs used simultaneously, and other medical factors.

In the present invention, the pharmaceutical composition may be characterized in the form of capsules, tablets, granules, injections, ointments, powders or beverages, and the pharmaceutical composition may be characterized by targeting humans. The pharmaceutical composition is not limited thereto, but may be formulated and used in the form of oral formulations, such as powders, granules, capsules, tablets, aqueous suspensions, etc., external preparations, suppositories, and sterile injectable solutions according to a conventional method, respectively.

The pharmaceutical composition according to the present invention may include a pharmaceutically acceptable carrier. The pharmaceutically acceptable carrier may be used with a binder, a lubricant, a disintegrant, an excipient, a solubilizer, a dispersant, a stabilizer, a suspending agent, a pigment, a flavoring, and the like during oral administration, may be mixed and used with a buffering agent, a preservative, a painless agent, a solubilizer, an isotonic agent, a stabilizer, and the like in the case of injections, and may be used with a base, an excipient, a lubricant, a preservative, and the like in the case of topical administration. The formulations of the pharmaceutical composition of the present invention may be prepared variously in combination with the pharmaceutically acceptable carrier described above.

For example, for oral administration, the pharmaceutical composition may be formulated in the form of tablets, troches, capsules, elixirs, suspensions, syrups, wafers, etc., and for injections, the pharmaceutical composition may be formulated into a single dose ampoule or a multiple dose form. In addition, the pharmaceutical composition may be formulated into solutions, suspensions, tablets, capsules, sustained release agents, and the like.

Meanwhile, examples of the carrier, the excipient, and the diluent suitable for the formulations may be used with lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils, or the like. In addition, the pharmaceutical composition may further include fillers, anti-coagulating agents, lubricants, wetting agents, flavorings, emulsifiers, preservatives, and the like.

The route of administration of the pharmaceutical composition according to the present invention is not limited thereto, but includes oral, intravenous, intramuscular, intraarterial, intramedullary, intrathecal, intracardiac, transdermal, subcutaneous, intraperitoneal, intranasal, intestinal, topical, sublingual or rectal administration. The "parenteral" includes subcutaneous, intradermal, intravenous, intramuscular, intraarticular, intrasynovial, intrasternal, intrathecal, intralesional and intracranial injection or infusion techniques. The pharmaceutical composition according to the present invention may also be administered in the form of a suppository for rectal administration.

The pharmaceutical composition according to the present invention may be variously changed according to various factors, including the activity of a specific active ingredient used, age, body weight, general health, sex, diet, administration time, administration route, excretion rate, drug combination, and the severity of a specific disease to be prevented or treated. The dose of the pharmaceutical composition varies depending on the condition and body weight of a patient, degree of a disease, drug form, and administration route and period, but may be appropriately selected by those skilled in the art. Preferably, the pharmaceutical composition may be administered with an amount capable of obtaining a maximum effect with a minimum amount without side effects in consideration of all the factors, and may be repeatedly administered in an effective amount of more preferably 1 to 10000 µg/weight kg/day, and much more preferably 10 to 1000 mg/weight kg/day several times a day. The dose does not limit the scope of the present invention in any aspect.

The pharmaceutical composition of the present invention may be used alone or in combination with surgery, radiation therapy, hormone therapy, chemotherapy, and methods of using biological response modifiers, for prevention or treatment of target indications.

Further, according to another aspect of the present invention, the present invention provides an anticancer adjuvant including a SET Domain and Mariner Transposase Fusion Gene (SETMAR) inhibitor.

As used herein, the "anticancer adjuvant" refers to an agent that can alleviate, improve, or increase the anticancer effect of the anticancer agent by administering the anticancer adjuvant in combination with the anticancer agent when administering the anticancer agent. In the present invention, the anticancer adjuvant may be used as an anticancer agent or anticancer adjuvant depending on a treatment concentration, and may enhance the sensitivity of the anticancer agent.

The adjuvant of the present invention may be administered simultaneously, separately or sequentially with or from the anticancer drug. The order of administration of the anticancer adjuvant according to the present invention, that is, which of the anticancer agent and the anti-cancer adjuvant is administered at some point and simultaneously, individually or sequentially, may be determined by a doctor or expert. The order of administration may vary depending on many factors. The anticancer adjuvant may be administered in combination with known compounds that have the effect of preventing, alleviating, or treating cancer. In this regard, the anticancer adjuvant may be administered simultaneously or sequentially with known compounds.

The known compound may be at least one anticancer agent selected from the group consisting of oxaliplatin, 5-fluorouracil (5-FU), doxorubicin, irinotecan, carboplatin, paclitaxel, gemcitabine and bortezomib, but is not limited thereto.

According to another aspect of the present invention, the present invention provides a food composition for preventing or alleviating cancer, including a SET Domain And Mariner Transposase Fusion Gene (SETMAR) inhibitor.

The food composition according to the present invention includes all forms, such as functional food, nutritional supplements, health food, health supplements, and food additives. The type of food composition may be formulated in any one form selected from the group consisting of powders, tablets, capsules, pills, and liquids according to a conventional method known in the art, but is not limited thereto. The food composition may be formulated in various forms using methods known in the art.

For example, as the health food, the SETMAR inhibitor itself of the present invention may be ingested by granulation, encapsulation and powder or prepared in the form of tea, juice, and drinks to be drunk. In addition, the SETMAR inhibitor of the present invention may be mixed with a known substance or active ingredient known to have the prevention, alleviation, or treatment activity of cancer to be prepared in the form of a composition.

In addition, the functional food may be prepared by adding the SETMAR inhibitor of the present invention to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., Udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc.

In addition, the food composition of the present invention may include conventional food additives, and the suitability as the "food additive" is determined by the specifications and standards for the corresponding item in accordance with the general rules of the Food Additive Codex, general test methods, and the like approved by the Food and Drug Administration, unless otherwise specified. The items disclosed in the "Food Additives Codex" may include, for example, chemical composites such as ketones, glycine, calcium citrate, nicotinic acid, cinnamic acid, etc., natural additives such as desensitizing dye, licorice extract, crystal cellulose, Kaoliang color, guar gum, etc., and mixed formulations such as sodium L-glutamic acid formulations, alkali agents for noodles, preservative formulations, tar color formulations, etc.

In the food composition of the present invention, the SETMAR inhibitor may be included preferably in an amount of 0.00001 to 50 wt% based on the food composition. If the content is less than 0.00001 wt%, the effect is insignificant, and if the content exceeds 50 wt%, an increase in effect compared to the amount used is minimal, which is uneconomical.

In addition, in order to use the SETMAR inhibitor of the present invention in the form of a food additive, the SETMAR inhibitor may be prepared and used in the form of tablets, capsules, powders, granules, liquids, pills, etc.

When the composition of the present invention is prepared as beverages, like general beverages, the composition may include various flavoring agents or natural carbohydrates as an additional ingredient. The above-mentioned natural carbohydrates may be used with monosaccharides such as glucose and fructose, disaccharides such as maltose and sucrose, natural sweeteners such as dextrin and cyclodextrin, synthetic sweeteners such as saccharin and aspartame, and the like. A ratio of the natural carbohydrates may be generally about 0.01 to 10 g, preferably about 0.01 to 0.1 g per 100 ml of the composition of the present invention.

In addition, the composition of the present invention may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohols, a carbonic acid agent used in a carbonated drink, or the like. In addition, the composition of the present invention may include pulps for preparing natural fruit juices, fruit juice beverages or vegetable beverages. These ingredients may be used independently or in combination. Although the ratio of these additives is not greatly important, generally, the ratio thereof is selected in a range of 0.01 to 0.1 parts by weight per 100 parts by weight of the composition of the present invention.

As used herein, the "health supplement" or "health functional food" refers to food prepared and processed using raw materials or ingredients with functionality, which are useful for the human body according to the Health Functional Foods Act, and the "functionality" means intake for adjusting nutrients for the structures and functions of the human body or obtaining a useful effect on health applications such as physiological actions.

According to yet another aspect of the present invention, the present invention provides a composition for inducing conversion of cancer cells into normal cells or normal-like cells including a SET Domain and Mariner Transposase Fusion Gene (SETMAR) inhibitor and a method for inducing conversion of cancer cells into normal cells or normal-like cells including treating cancer cells with the SETMAR inhibitor *in vitro.*

According to yet another aspect of the present invention, the present invention provides a screening method of a cancer therapeutic agent including the following steps:
(a) treating cancer cells with a candidate substance;
(b) measuring the expression level of SETMAR in the cancer cells treated with the candidate substance; and
(c) determining the candidate substance as a cancer therapeutic agent if the expression level of SETMAR is lower than that of a control group untreated with the candidate substance.

According to the screening method of the present invention, a candidate substance to be analyzed may first be in contact with cancer cells including the gene or protein. The candidate substance refers to an unknown substance used in screening to test whether the candidate substance affects the expression level of the gene, the amount of protein, or the activity of the protein.

The candidate substance may include, but are not limited to, chemicals, antisense oligonucleotides, antisense-RNA, siRNA, shRNA, miRNA, antibodies specific for the protein, or natural product extracts.

In the present invention, the candidate substance may include siRNA, shRNA, miRNA, or antibodies specific for SETMAR.

Thereafter, the expression level of the gene, the amount of the protein, or the activity of the protein may be measured in the cells treated with the candidate substance. As the measured result, when it is measured that the expression level of the gene, the amount of the protein, or the activity of the protein is decreased, it may be determined that the candidate substance may be used as an agent capable of treating or preventing cancer.

The method of measuring the expression level of the gene or the amount of protein may be performed including a known process of isolating mRNA or protein from a biological sample using a known technique. The "biological sample" refers to a sample collected from a living body of which the gene expression level or protein level is different from that of the control group. Examples of the sample may include tissue, cells, blood, serum, plasma, saliva, and urine, but are not limited thereto.

The expression level of the gene is preferably measured by measuring the level of mRNA, and methods for measuring the mRNA level include reverse transcription polymerase chain reaction (RT-PCR), real-time reverse transcription polymerase chain reaction, RNase protection assay, Northern blot, DNA chips, etc., but are not limited thereto.

The protein level may be measured using an antibody. In this case, the marker protein in the biological sample and a specific antibody thereto form a combination, that is, an antigen-antibody complex, and the formed amount of the antigen-antibody complex may be measured quantitatively through the signal size of a detection label. The detection label may be selected from the group consisting of enzymes, fluorescent substances, ligands, luminescent substances, microparticles, redox molecules, and radioisotopes, but is not limited thereto. Analytical methods for measuring the protein levels include Western blot, ELISA, radioimmunoassay, radioimmunodiffusion, Ouchterony immunodiffusion, rocket immunoelectrophoresis, tissue immunostaining, immunoprecipitation assay, complement fixation assay, FACS, protein chips, etc., but are not limited thereto.

According to yet another aspect of the present invention, the present invention provides a screening method of an agent capable of converting cancer cells into normal cells or normal-like cells, including the following steps:
(a) treating cancer cells with a candidate substance;
(b) measuring the expression level of SETMAR in the cancer cells treated with the candidate substance; and
(c) determining the candidate substance as an agent capable of converting the cancer cells into the normal cells or normal-like cells if the expression level of SETMAR is lower than that of a control group untreated with the candidate substance.

According to yet another aspect of the present invention, the present invention provides a method for treating cancer including administering a SET Domain and Mariner Transposase Fusion Gene (SETMAR) inhibitor to a subject.

As used herein, the term "subject" refers to an individual having a cancer disease, preferably mammals such as horse, sheep, pig, goat, dog, including humans having the cancer disease, but preferably human.

Since the method of the present invention uses the SETMAR inhibition described above, the description of duplicated contents will be omitted to avoid excessive complexity of the present specification.

### [Advantageous Effects]

According to the present invention, it has been identified that if SETMAR is inhibited in cancer cells, carcinogenesis reversion, which is the differentiation of cancer cells into normal cells, is successfully achieved, and thus, unlike conventional anticancer agents that simply induce cancer cell death, the present invention can be effectively used as a treatment method excluding the side effect of normal cell death that can occur during anticancer treatment, and converting cancer cells into normal cells.

### [Description of Drawings]

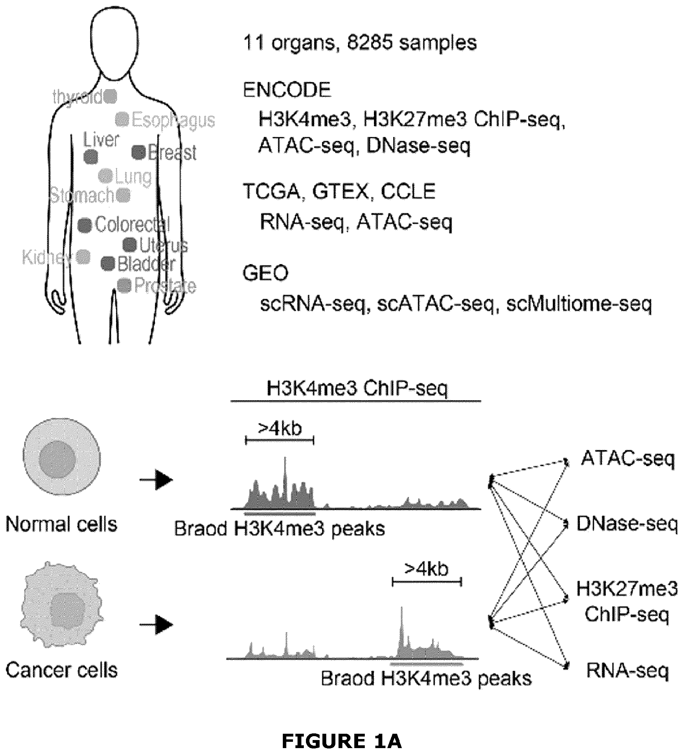

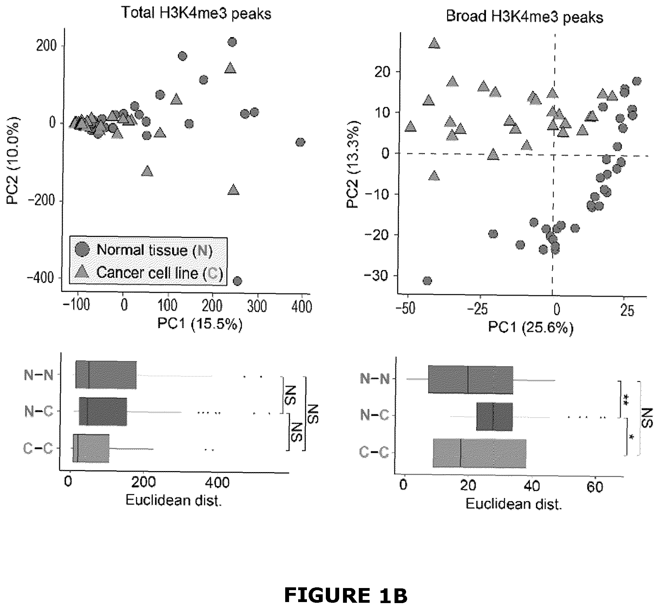

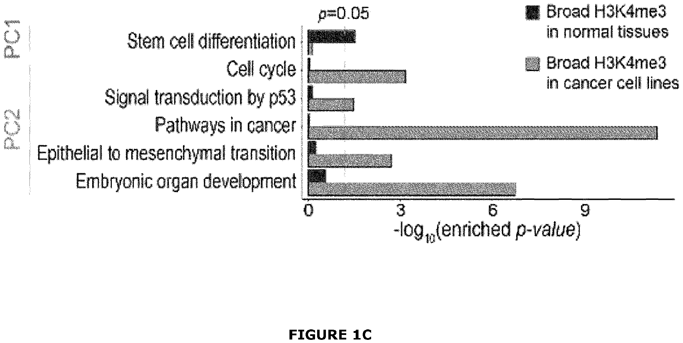

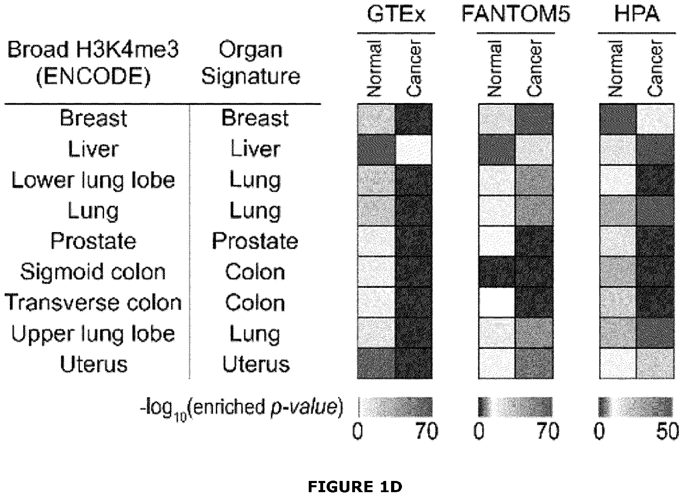

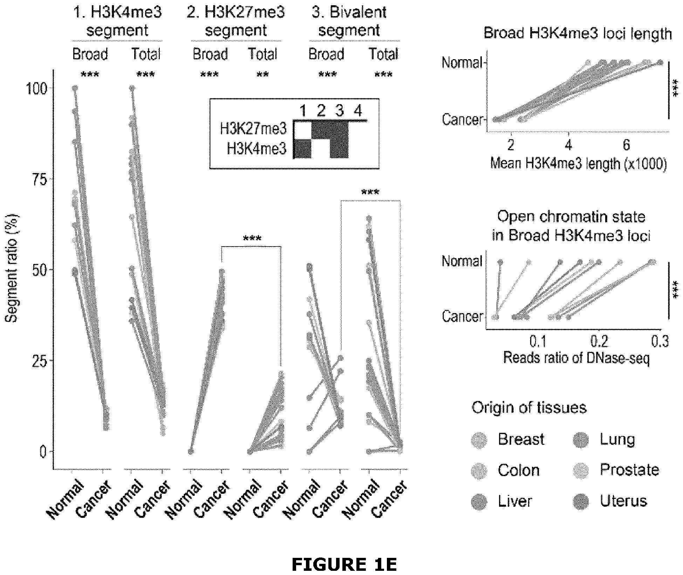

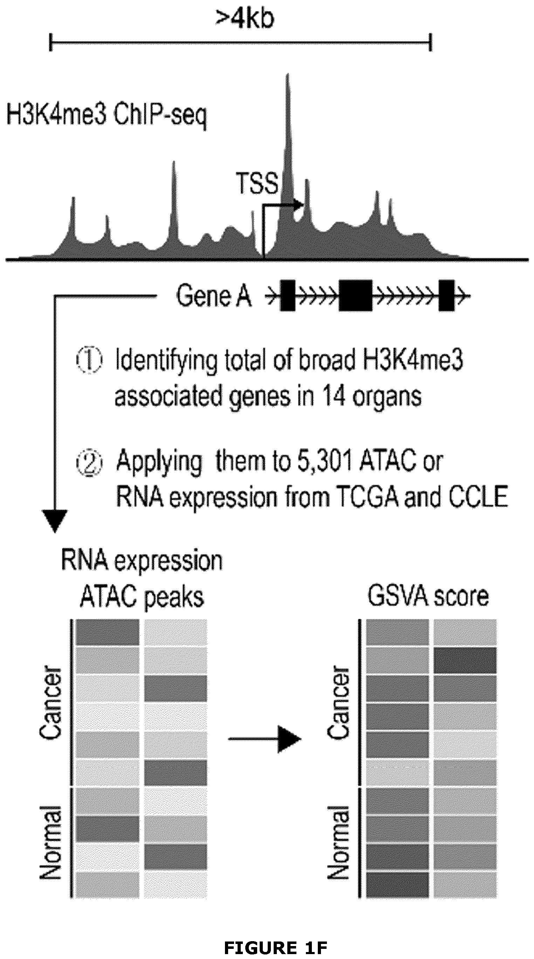

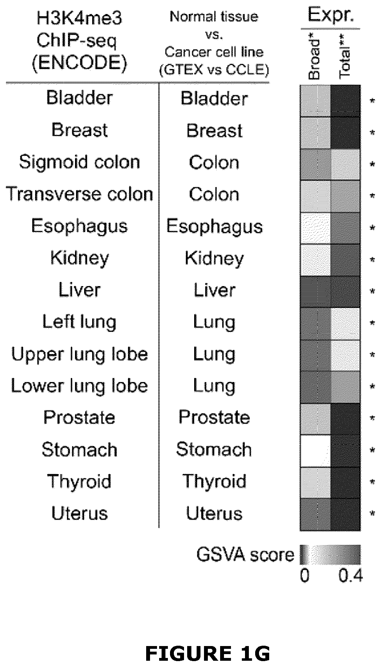

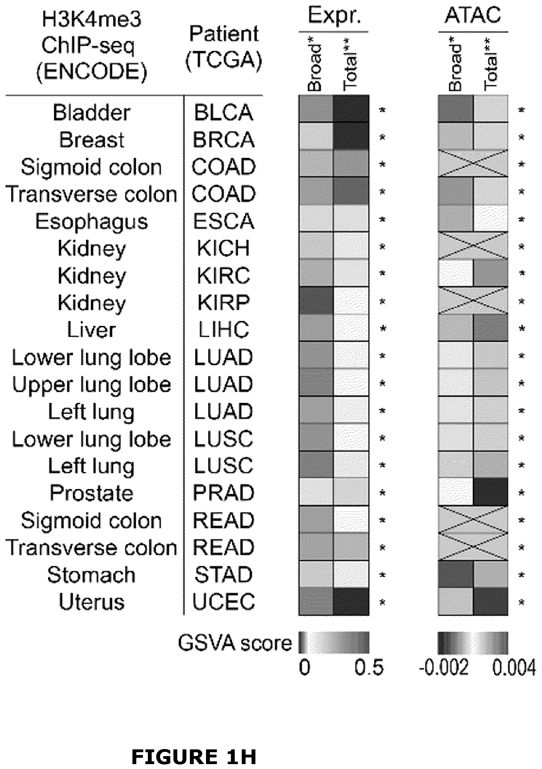

FIGS. 1A to 1H illustrate results of confirming that epigenetic regulatory genes important in carcinogenesis have statistically significant correlations through data analysis in various tissues. FIG. 1A schematically shows results of deriving H3K4me3 Peaks (Broad H3K4me3) of 4 kb or more indicating differences between cancer cells and normal cells based on a known database. FIG. 1B shows results of principal component analysis (PCA) for H3K4me3 data derived from a normal tissue and H3K4me3 data derived from a cancer cell line based on Total H3K4me3 Peaks and Broad H3K4me3 Peaks. FIG. 1C shows a result of gene ontology (GO) analysis using main genes constituting an axis of PCA. FIG. 1D shows results of comparing genes associated with Broad H3K4me3 Peaks using previously known organ-specific gene databases GTEx, FANTOMS, and HPA, respectively. FIG. 1E shows results of identifying the presence or absence of H3K4me3 and/or H3K27me3 by analyzing the entire human genome by 10 kb, and comparing changes in Broad H3K4me3 Peaks and Total H3K4me3 Peaks in normal and cancer tissues based thereon. FIG. 1F shows a method of selecting locations and associated genes of Total and Broad H3K4me3 and identifying how much a transcriptome and an epigenome have been increased. FIG. 1G shows results of integrating a normal tissue transcriptome database GTEX and a cancer cell line transcriptome database, and comparing the expression levels of previously selected Broad H3K4me3 associated genes in the two databases. At this time, the Broad H3K4me3 associated genes differ significantly between cancer and normal cells. FIG. 1H shows results of summarizing changes in the transcriptomes and epigenomes of normal tissues and cancer cells in the TCGA database, and comparing the expression levels of transcriptomes and the changes in the epigenomes of the Broad H3K4me3 associated genes previously selected from the databases. At this time, the Broad H3K4me3 associated genes differ significantly between cancer and normal transcriptomes and epigenomes.
FIG. 2 shows results of discovering important epigenetic regulator targets in the carcinogenesis by analyzing epigenetic regulators capable of directly binding to and regulating Broad H3K4me3, which is known to be important in normal tissues, at a pan-cancer level.
FIGS. 3A and 3B show results of identifying proliferation inhibition and phenotypic changes to normal cells of SETMAR-inhibited cancer cells. KD; knock-down
FIGS. 4A and 4B show results of identifying expression levels of normal-associated genes and proteins thereof in SETMAR-inhibited cancer cells.
FIG. 5 shows results of identifying recovery of normal cellular metabolic functions in SETMAR-inhibited cancer cells.
FIG. 6 shows results of identifying reduction of metastatic ability in SETMAR-inhibited cancer cells.
FIGS. 7A and 7B show results of identifying various epigenetic changes in cancer cells affected by SETMAR inhibition.
FIG. 8 shows results of identifying the effects of SETMAR inhibition on single-cell transcriptomes and epigenomes in cancer cells.
FIG. 9 shows a result of comparing cancer cell growths in a xenograft model in which a liver cancer cell line with SETMAR as a comparative control group and a liver cancer cell line of the present invention in which SETMAR is inhibited are transplanted into nude mice.

### [Modes of the Invention]

Hereinafter, Examples are to describe the present invention in more detail, and it will be apparent to those skilled in the art that the scope of the present invention is not limited by these Examples in accordance with the gist of the present invention.

### Experimental materials and methods

All liver cancer cell lines SNU-475 and SNU-761 used in the present invention were provided by the Seoul National University Cell Bank, and 10% FBS and DMEM from Welgene were used. The cells were cultured at 37°C in a humidified atmosphere with 5% CO₂, and all conditions followed the protocol of the Seoul National University Cell Line Bank, where the cell line was established.

To inhibit SETMAR in the cell lines, shRNA (TCCGACTCCAATTACATTATA, SEQ ID NO: 1) was delivered into cells using Lenti virus to inhibit the expression of SETMAR. In addition, the reduction thereof was identified through RT-qPCR using SETMAR-F (GGATGGCGGAGTTTAAGGAGA, SEQ ID NO: 2) and SETMAR-R (GCTGGGTTCCTTCTCATTTCC, SEQ ID NO: 3) primers.

Various sequencing and Omics data used in the present invention followed the manufacturer's pipeline. ChIP-seq followed the protocol of ChIP-IT high sensitive from Activie motif, and all analyses followed the Bowtie-MACS2 pipeline. In the case of single cell RNA-seq, data was produced using a single cell multiome kit from 10x Genomics, and then aligned at hg38 using a Cellranger-arc program, and Seurat, Signac, and FigR were used for subsequent analysis.

In the present invention, animal experiments were conducted by a method approved by the Institutional Animal Care and Use Committee of the Korea Advanced Institute of Science and Technology, and Xenograft mice were produced by transplanting cancer cells under the skin of mice, and a therapeutic effect of SETMAR was confirmed through this model.

### Example 1. Discovery of epigenetic regulator targets in carcinogenesis

The present inventors conducted data analysis constituting a gene regulatory network, based on genome, epigenome, and transcriptome databases TCGA, ENCODE, GTEX, HPA, and FANTOM of various cancer and normal tissues to discover important epigenetic regulator targets in the carcinogenesis of cancer.

First, as shown in FIGS. 1A to 1H, 8,000 samples or more from 11 types of organs were integrated and analyzed. H3K4me3 peaks of 4 kb or more (Broad H3K4me3 Peaks) that showed differences between normal and cancer cells were identified and it was confirmed that genes regulated by these peaks had a statistically significant correlation with genes that regulated major functions of each organ.

That is, Broad H3K4me3 Peaks were obtained by integrated analysis of various data from 8,000 or more samples (FIG. 1A), and when it was confirmed whether normal cells and cancer cells were distinguished using Total histone modification loci and Broad H3K4me3 histone modification markers of 4 kb or more, it was impossible to distinguish normal cells and cancer cells using Total histone modification loci (Total H3K4me3 Peaks), whereas it was confirmed that Broad H3K4me3 histone modification (Broad H3K4me3 Peaks) could effectively distinguish the normal cells and cancer cells (FIG. 1B). As a result of identifying genes corresponding to a main principal component (PC) axis by analyzing main components based on the result, it was confirmed that PC1 was mainly related to the differentiation of normal cells, and PC2 was mainly related to the characteristics of cancer cells (FIG. 1C). In addition, as a result of identifying genes associated with the Broad H3K4me3 loci using GTEx, FANTOMS, and HPA data, it was found that the genes matched the characteristics of each organ (FIG. 1D). In addition, it was shown that while the lengths of the Broad H3K4me3 loci of normal tissues were significantly reduced in cancer cells, the characteristics of H3K27me3 were acquired (FIG. 1E). In addition, in addition to the comparison of the normal cells and the cancer cell lines, as a result of additionally confirming that the expression of genes associated with Broad H3K4me3 and Total H3K4me3 and epigenetic state changes in normal tissues using TCGA, GTEx, and CCLE cancer data, in Total H3K4me3, there was a small difference in expression levels between cancer cells and normal cells, whereas in Broad H3K4me3, there was a large difference in expression levels between cancer cells and normal cells (FIGS. 1F to 1H).

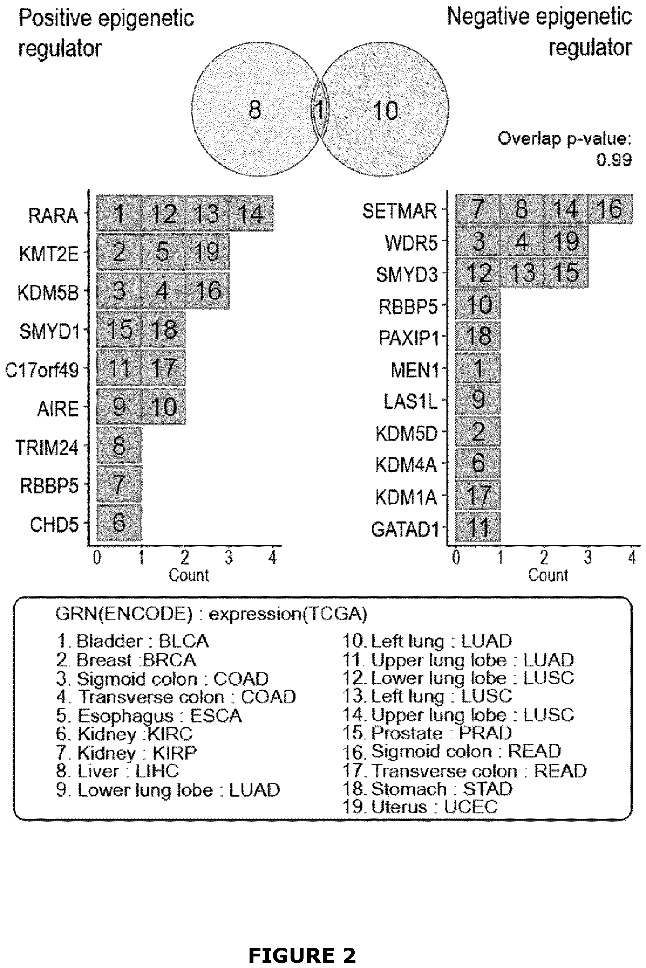

In addition, as shown in FIG. 2, as a result of analyzing epigenetic regulators capable of directly binding to and regulating Broad H3K4me3 known to be important in normal tissues at a pan-cancer level, a SET Domain And Mariner Transposase Fusion Gene (SETMAR) was finally selected as a master epigenetic regulator that led reversion (differentiation) of liver cancer, colon cancer, lung cancer, and kidney cancer cells into normal cells at the pan-cancer level.

Hereinafter, in Example, the experiment was conducted by representatively targeting liver cancer.

### Example 2. Effects of inhibition of epigenetic regulator SETMAR of the present invention on cancer cells

### 2-1. Confirmation of proliferation inhibition and induction of phenotypic changes to normal cells of SETMAR-inhibited cancer cells

In order to confirm the effect of inhibition of SETMAR, the epigenetic regulator selected in Example 1, on cancer cells, the present inventors produced two types of liver cancer cell lines SNU475 and SNU761 with reduced SETMAR using shRNA, and observed the proliferation levels and shapes of the cancer cell lines.

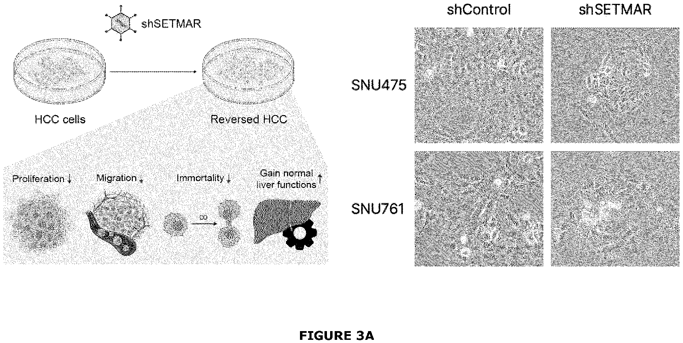

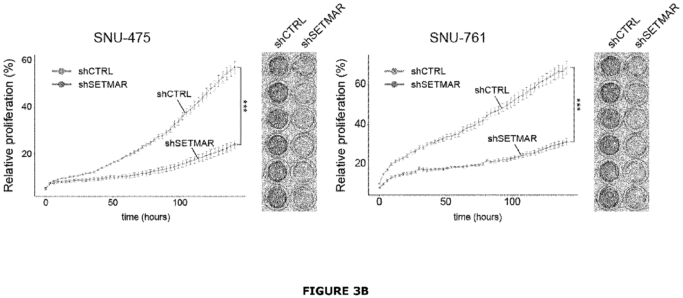

As a result, as shown in FIG. 3A, it was confirmed that the cells reversed by SETMAR had a cell shape in which the original long and pointed phenotypic characteristics disappeared and a phenotype was changed to a round and short normal hepatocyte shape. In addition, as shown in FIG. 3B, the proliferation of both cell lines was significantly reduced.

These results show that SETMAR inhibition does not simply kill cancer cells, but may also reverse cancer cells into normal cells to have phenotypic characteristics of normal cells.

### 2-2. Induction of increased expression of normal-related genes in SETMAR-inhibited cancer cells

In addition, to verify whether the liver cancer cells were actually reversed into normal cells (or normal-like cells), the present inventors confirmed the expression levels of albumin and major hepatocyte function-related genes and proteins, as markers known to be important in normal cells, in the SETMAR-inhibited liver cancer cell lines SNU475 and SNU761.

The major hepatocyte function-related genes were as follows:
AAT : Alpha 1 anti-trypsin, ALB : Albumin, ALDOB : Aldolase, Fructose-Bisphosphate B, CYP1A2 : Cytochrome P450 1A2, G6P : Glucose 6-phosphate, GS : Glutamine synthetase, MRP2 : Multidrug Resistance-Associated Protein 2, PCK1 : Phosphoenolpyruvate Carboxykinase 1, PEPCK : Phosphoenolpyruvate Carboxykinase, ASGR2 : Asialoglycoprotein Receptor 2, CEBPA : CCAAT Enhancer Binding Protein Alpha, ONECUT1 (HNF6A) : One Cut Homeobox 1. TF : Transferrins, LIPC : Hepatic lipase, C3 : Complement subunit 3, ASGR1 : glycoprotein that forms the asialoglycoprotein receptor, FOXA3 : Forkhead Box A3.

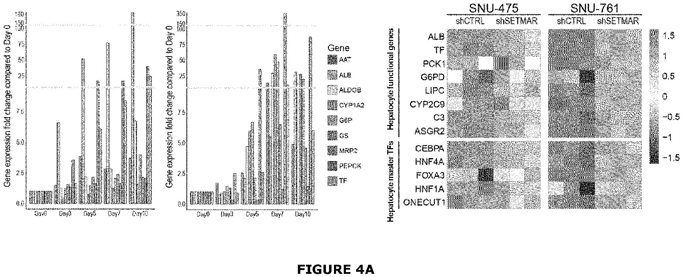

As a result, as shown in FIG. 4A, in the liver cancer cell line in which SETMAR of the present invention was inhibited and reversed into normal hepatocytes, as a result of confirming changes in the expression levels of the genes known to be related to the function of normal hepatocytes by qPCR, it was confirmed that these genes increased at least 2-fold, and albumin increased up to 100-fold. The albumin is one of various essential proteins produced by hepatocytes, and when liver failure or liver cancer occurs and albumin production is suppressed, the osmotic pressure in the body is broken and ascites are filled. Accordingly, the production of the albumin means that normal liver functions are exhibited.

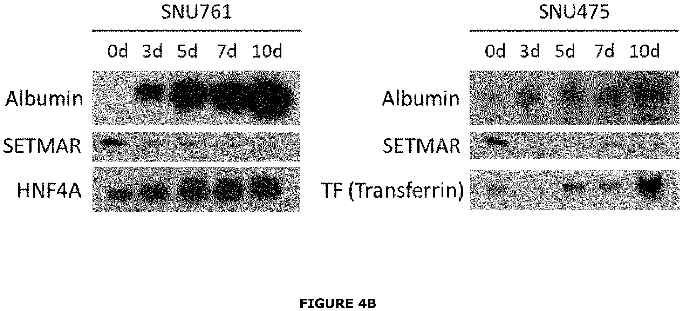

In addition, as shown in FIG. 4B, it was confirmed that the expression level of the albumin protein also increased significantly.

### 2-3. Induction of recovery of normal cellular metabolic functions in SETMAR-inhibited cancer cells

In addition, to verify whether the liver cancer cells were actually reversed into normal cells (or normal-like cells), the present inventors measured the recovery level of normal hepatocyte metabolic functions in the SETMAR-inhibited liver cancer cell line.

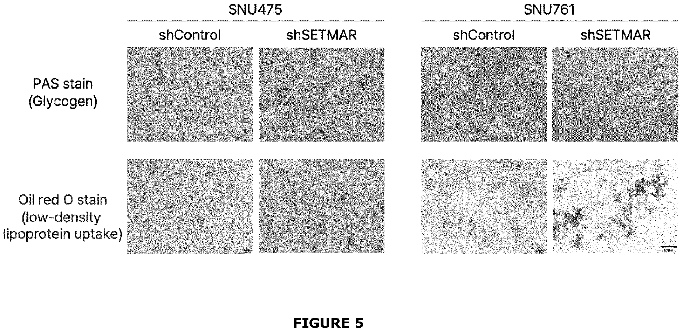

As a result, as shown in FIG. 5, the recovery of liver functions of liver cancer cells due to the decrease in SETMAR was confirmed by the increase in albumin, and further, through PAS related to glucose metabolism and Oil red O staining related to lipid metabolism, it was confirmed that the actual hepatocyte metabolic functions were recovered.

### 2-4. Induction of reduced metastatic ability in SETMAR-inhibited cancer cells

The present inventors observed the effect of inhibition (reduction) of SETMAR on the metastatic ability of cancer cells.

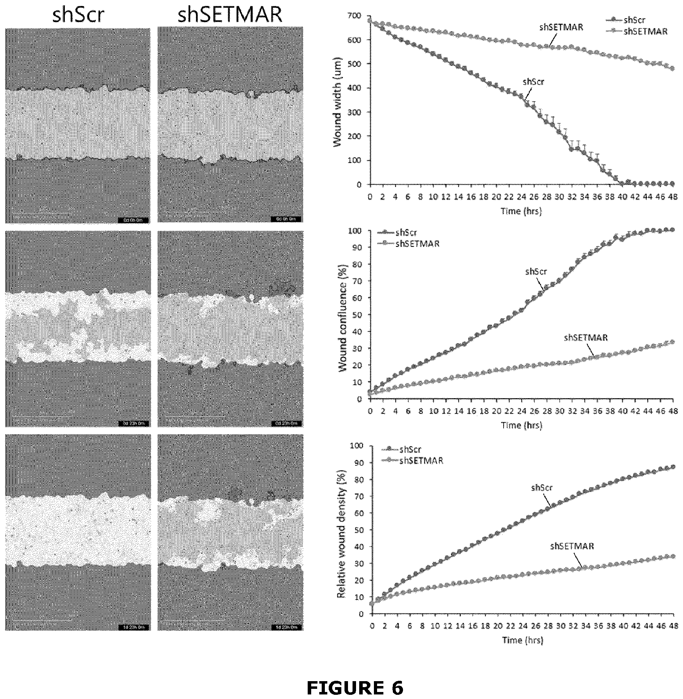

As a result, as shown in FIG. 6, it was confirmed that inhibition of SETMAR significantly reduced the metastatic ability in the SNU475 cell line actually having the metastatic ability.

### Example 3. Various epigenetic changes in cancer cells caused by inhibition of epigenetic regulator SETMAR of the present invention

The present inventors observed the effect of inhibition of SETMAR as the selected epigenetic regulator of the present invention on epigenetic changes in cancer cells.

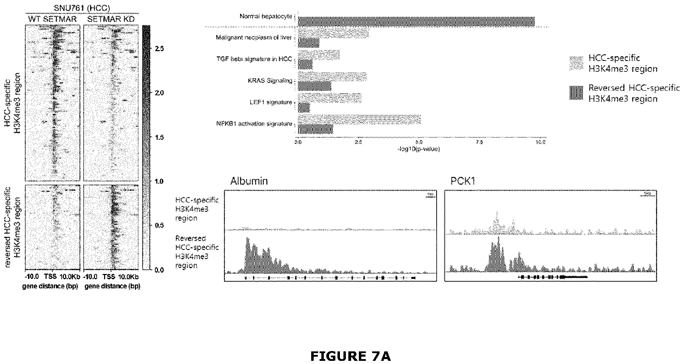

As a result, the inhibition of SETMAR induced various epigenetic changes, and as shown in FIG. 7A, a change (increase) in H3K4me3 was induced actually, and the increase in H3K4me3 was mainly observed in albumin and phosphoenolpyruvate carboxykinase, which were important genes related to hepatocyte functions.

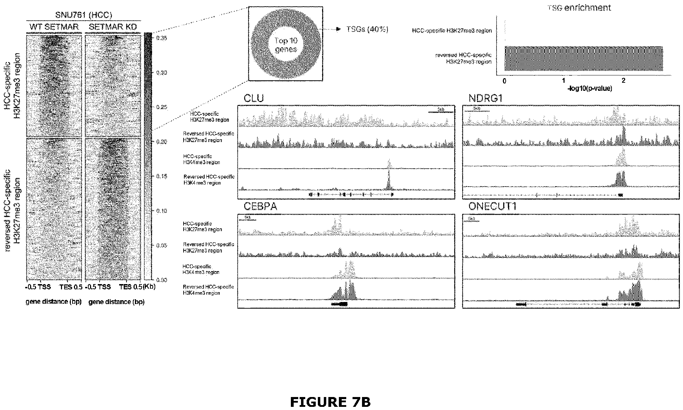

In contrast, as shown in FIG. 7B, the inhibition of SETMAR induced a decrease in H3K27me3, which was mainly observed in tumor suppressor genes.

The results verified that the SETMAR inhibition of the present invention not only increased histone H3 trimethylation at lysine 4 (H3K4me3), which was a characteristic of normal cells, but also induced an epigenetic change in which histone H3 trimethylation at lysine 27 (H3K27me3), which was a characteristic of tumor suppressor genes, was inhibited, so that conversion, that is, reversion of cancer cells into normal cells occurred.

### Example 4. Effects of inhibition of epigenetic regulator SETMAR of the present invention on single-cell transcriptome and epigenome in cancer cells

The present inventors analyzed data to confirm the effects of inhibition of SETMAR as the selected epigenetic regulator of the present invention on the single-cell transcriptome and epigenome of cancer cells.

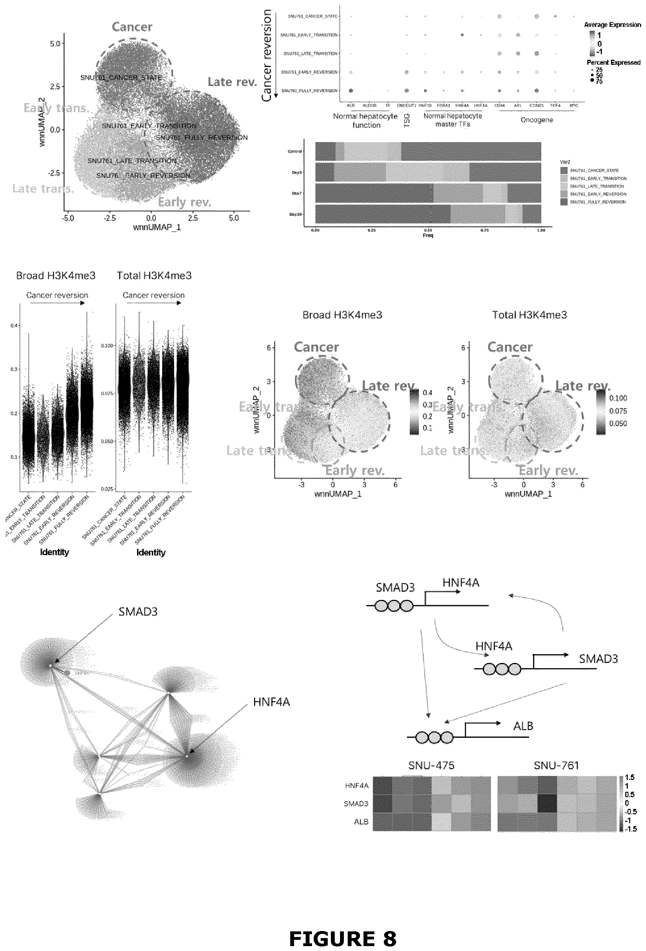

As a result, as shown in FIG. 8, it was confirmed that after the SETMAR was actually inhibited in liver cancer cells, the cancer cells were reversed into normal cells over time, that is, albumin increased in the same manner as in normal hepatocytes, and simultaneously, HNF4A, FOXA1, and HNF1A, which were important transcription factors in normal liver cells, increased.

### Example 5. Confirmation of cancer cell inhibition effect in vivo by inhibition of epigenetic regulator SETMAR of the present invention

The present inventors intended to confirm the growth level of cancer cells according to SETMAR inhibition *in vivo.*

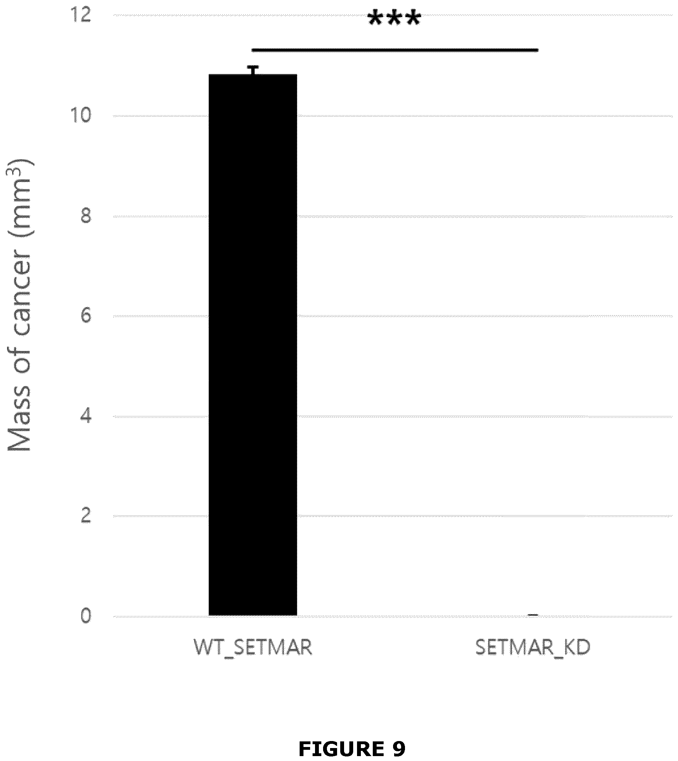

Accordingly, as a result of measuring and comparing sizes of these cancer cells on day 7, after transplanting HCC (SNU761) (WT_SETMAR) expressing normal SETMAR as a control group and SETMA-knockdown HCC (SETMAR_KD) of the present invention into nude mice, as shown in FIG. 9, unlike a SETMAR-expressing HCC treated group that formed tumors, in a SETMAR-inhibited HCC treated group, it was confirmed that not only cancer was not formed, but also proliferation did not occur at all (***p-value < 0.001).

It is verified that the cancer cell line not only does not cause cancer, but also is reversed into normal cells by losing the unique characteristics as a cancer cell by SETMAR inhibition, thereby achieving an ultimate goal of complete remission of cancer, i.e., a state where no cancer cells exist, i.e., only normal cells or normal-like cells exist.

Therefore, the SETMAR inhibition of the present invention may effectively act in anticancer treatment.

In summary, in the present invention, it was verified that the inhibition of SETMAR not only simply inhibited cancer characteristics, but also changed cancer cells to express characteristics of normal cells, thereby exhibiting the phenotype and functions of normal cells. Therefore, it is expected that it is possible to achieve safe and effective anticancer treatment effects by reversing cancer cells into normal cells through control of these epigenetic regulators.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A pharmaceutical composition for preventing or treating cancer, comprising a SET Domain and Mariner Transposase Fusion Gene (SETMAR) inhibitor as an active ingredient.

2. The pharmaceutical composition for preventing or treating cancer of claim 1, wherein the SETMAR inhibitor is at least one selected from the group consisting of antisense oligonucleotide, small interference RNA (siRNA), short hairpin RNA (shRNA), microRNA (miRNA), and ribozyme that bind complementarily to mRNA of a SETMAR gene.

3. The pharmaceutical composition for preventing or treating cancer of claim 1, wherein the SETMAR inhibitor is at least one selected from the group consisting of a compound, a peptide, a peptide mimetic, a substrate analog, an aptamer, and an antibody that specifically bind to a SETMAR protein.

4. The pharmaceutical composition for preventing or treating cancer of claim 1, wherein the SETMAR inhibitor induces reversion of cancer cells into normal cells or normal-like cells.

5. The pharmaceutical composition for preventing or treating cancer of claim 4, wherein the reversion into the normal cells or normal-like cells induces a change to normal cell shape, recovery of normal cell functions, or epigenetic changes to normal cells.

6. The pharmaceutical composition for preventing or treating cancer of claim 1, wherein the SETMAR inhibitor inhibits at least one selected from the group consisting of proliferation ability, growth ability, metastatic ability, invasion ability, and migration ability of cancer cells.

7. The pharmaceutical composition for preventing or treating cancer of claim 1, wherein the SETMAR inhibitor increases or inhibits methylation of histones in cancer cells.

8. The pharmaceutical composition for preventing or treating cancer of claim 7, wherein the SETMAR inhibitor increases trimethylation of histone H3 at lysine 4 (H3K4me3).

9. The pharmaceutical composition for preventing or treating cancer of claim 7, wherein the SETMAR inhibitor inhibits trimethylation of histone H3 at lysine 27 (H3K27me3).

10. The pharmaceutical composition for preventing or treating cancer of claim 1, wherein the cancer is at least one selected from the group consisting of liver cancer, colon cancer, lung cancer, adrenal cancer, stomach cancer, breast cancer, blood cancer, bone cancer, pancreatic cancer, skin cancer, head or neck cancer, cutaneous or intraocular melanoma, uterine sarcoma, ovarian cancer, rectal cancer, anal cancer, fallopian tube carcinoma, endometrial carcinoma, cervical cancer, small intestine cancer, endocrine cancer, thyroid cancer, parathyroid cancer, soft tissue tumor, urethral cancer, prostate cancer, bronchogenic cancer, and bone marrow tumor.

11. An anticancer adjuvant comprising a SET Domain and Mariner Transposase Fusion Gene (SETMAR) inhibitor as an active ingredient.

12. A food composition for preventing or alleviating cancer, comprising a SET Domain And Mariner Transposase Fusion Gene (SETMAR) inhibitor as an active ingredient.

13. A composition for inducing conversion of cancer cells into normal cells or normal-like cells comprising a SET Domain and Mariner Transposase Fusion Gene (SETMAR) inhibitor as an active ingredient.

14. A method for inducing conversion of cancer cells into normal cells or normal-like cells comprising treating the cancer cells with a SET Domain and Mariner Transposase Fusion Gene (SETMAR) inhibitor in *vitro.*

15. A screening method of a cancer therapeutic agent comprising following steps:
(a) treating cancer cells with a candidate substance;
(b) measuring an expression level of SETMAR in the cancer cells treated with the candidate substance; and
(c) determining the candidate substance as the cancer therapeutic agent if the expression level of SETMAR is lower than that of a control group untreated with the candidate substance.

16. A screening method of an agent capable of converting cancer cells into normal cells or normal-like cells, comprising following steps:
(a) treating the cancer cells with a candidate substance;
(b) measuring an expression level of SETMAR in the cancer cells treated with the candidate substance; and
(c) determining the candidate substance as the agent capable of converting the cancer cells into the normal cells or normal-like cells if the expression level of SETMAR is lower than that of a control group untreated with the candidate substance.

17. A method for treating cancer comprising administering a SET Domain and Mariner Transposase Fusion Gene (SETMAR) inhibitor to a subject.
